# EUROPEAN PATENT APPLICATION

(11) **EP 1 402 893 A1**
(43) Date of publication of application: **31.03.2004**
(21) Application number: 02741394.7
(22) Date of filing: 02.07.2002
(51) Int. Cl.: A61K 31/593, A61P 5/18, A61P 9/10

(54) **PHARMACEUTICAL COMPOSITIONS FOR TREATMENT OF SECONDARY HYPERPARATHYROIDISM AND THERAPEUTIC AGENTS FOR CARDIOVASCULAR COMPLICATIONS RESULTING FROM THE TREATMENT FOR SECONDARY HYPERPARATHYROIDISM.**

(30) Priority: 03.07.2001 JP 2001201673
(71) Applicant: CHUGAI SEIYAKU KABUSHIKI KAISHA, Tokyo, 115-8543 (JP)
(72) Inventor: HIRATA, Michinori, c/o Chugai Seiyaku K. K., Gotenba-shi, Shizuoka 412-8513 (JP)
(74) Representative: VOSSIUS & PARTNER
(86) International application number: PCT/JP2002/006690
(87) International publication number: WO 2003/004036

(57) **Abstract**

An object of the present invention is to provide a pharmaceutical composition for the treatment of secondary hyperparathyroidism, which can reduce the risk of inducing cardiovascular complications associated with calcification, as well as a therapeutic or prophylactic agent for cardiovascular complications (e.g., arteriosclerosis, ischemic heart disease) associated with calcification resulting from the treatment of secondary hyperparathyroidism in a hemodialysis patient.

The present invention provides a pharmaceutical composition for the treatment of secondary hyperparathyroidism, which comprises, as an active ingredient, a vitamin D derivative not having an inducing action on cardiovascular complications associated with calcification, as well as a therapeutic agent for cardiovascular complications associated with calcification resulting from the treatment of secondary hyperparathyroidism in a hemodialysis patient, which comprises 1α,3β-dihydroxy-20S-(3-hydroxy-3-methylbutyloxy)-9,10-seco-5,7,10(19)-pregnatriene as an active ingredient.

## Description

### TECHNICAL FIELD

The present invention relates to a pharmaceutical composition for the treatment of secondary hyperparathyroidism, which comprises, as an active ingredient, a vitamin D derivative not having an inducing action on cardiovascular complications associated with calcification.

In addition, the present invention relates to a therapeutic or prophylactic agent for cardiovascular complications associated with calcification resulting from the treatment of secondary hyperparathyroidism in hemodialysis patients, which agent comprises 1α,3β-dihydroxy-20S-(3-hydroxy-3-methylbutyloxy)-9,10-seco-5,7,10(19)-pregnatriene (22-oxa-1α,25-dihydroxyvitamin D₃; hereinafter also referred to as "OCT") as an active ingredient.

### BACKGROUND ART

Vitamin D has been administered in the treatment of secondary hyperparathyroidism (hereinafter also referred to as "2HPT") (Drugs of the Future, 21(12), 1229-1237 (1996)). However, 1,25-dihydroxyvitamin D₃ has a risk of inducing cardiovascular calcification because of its relatively strong hypercalcemic effect (Am. J. Kidney Diseases, 29, 641-649, 1997). For this reason, there has been a need to develop a pharmaceutical composition having a reduced risk of inducing cardiovascular complications associated with calcification.

It is known that cardiovascular disorders account for nearly half the deaths in hemodialysis patients, most of who are found to have calcification in the heart and vascular system (Raggi P., Clinical Nephrology, vol. 54, No. 6, pp. 325-33, 2000). Causes of cardiovascular disorders include platelet aggregation, blood lipids (e.g., hyperlipidemia) and vascular calcification. Recent analyses using electron beam computed tomography (EBCT) have suggested the relationship between mitral valve calcification and heart disorders (Stehen G. et al., Kidney Int., vol. 56, pp. 383-392, 1999). It has also been reported that there is a positive relationship between vascular calcification and blood calcium x phosphorus product (Ca × Pi product) (Keigo Kimura et al., Kidney Int., vol. 56, suppl. 71, pp. S238-241, 1999). Thus, in hemodialysis patients, cardiovascular calcification associated with elevated blood Ca × Pi product may be one of the risk factors promoting heart diseases.

Treatment with vitamin D is effective for secondary hyperparathyroidism (hereinafter also referred to as "2HPT"), which is one of the complications in hemodialysis patients. However, 1,25-dihydroxyvitamin D₃ has the risk of inducing cardiovascular calcification because of its relatively strong hypercalcemic effect (Am. J. Kidney Diseases, 29, 641-649, 1997). For this reason, there has been a need to develop compounds having a weak hypercalcemic effect and a reduced risk of inducing ectopic calcification.

### DISCLOSURE OF THE INVENTION

An object of the present invention is to provide a pharmaceutical composition for the treatment of secondary hyperparathyroidism, which can reduce the risk of inducing cardiovascular complications associated with calcification.

As a result of extensive and intensive efforts, the inventors of the present invention have found that the problems stated above can be overcome by using a pharmaceutical composition, which comprises a vitamin D derivative having a weak elevating action on Ca × Pi product. This finding led to the provision of the present invention.

Namely, the present invention provides a pharmaceutical composition for the treatment of secondary hyperparathyroidism, which comprises, as an active ingredient, a vitamin D derivative not having an inducing action on cardiovascular complications associated with calcification. Also, the present invention provides a pharmaceutical composition for the treatment of secondary hyperparathyroidism, which comprises, as an active ingredient, a vitamin D derivative having a prophylactic or improving action on cardiovascular complications associated with calcification resulting from the treatment of secondary hyperparathyroidism. Examples of cardiovascular complications include arteriosclerosis and ischemic heart disease.

In these pharmaceutical compositions, the elevating action of each vitamin D derivative on Ca × Pi product is preferably weak. Moreover, the vitamin D derivative is preferably 1α,3β-dihydroxy-20S-(3-hydroxy-3-methylbutyloxy)-9,10-seco-5,7,10(19)-pregnatriene.

According to the present invention, there is also provided a pharmaceutical composition for the treatment of secondary hyperparathyroidism, which comprises a vitamin D derivative as an active ingredient and which is administered to a patient having the risk of developing cardiovascular complications associated with calcification.

According to another aspect of the present invention, there is provided a treatment method of secondary hyperparathyroidism, which comprises administering any one of the above pharmaceutical compositions to a patient in need of such treatment, preferably to a patient having the risk of developing cardiovascular complications associated with calcification.

According to yet another aspect of the present invention, there is provided the use of a vitamin D derivative not having an inducing action on cardiovascular complications associated with calcification for the manufacture of a medicament used for the treatment of secondary hyperparathyroidism.

An object of the present invention is to provide a therapeutic or prophylactic agent for cardiovascular complications (e.g., arteriosclerosis, ischemic heart disease) associated with calcification resulting from the treatment of secondary hyperparathyroidism in a hemodialysis patient.

As a result of extensive and intensive efforts made to overcome the problems stated above, the inventors of the present invention have found that 1α,3β-dihydroxy-20S-(3-hydroxy-3-methylbutyloxy)-9,10-seco-5,7,10(19)-pregnatriene does not cause increase in blood calcium levels of experimental nephritis model rats with 2HPT. This finding led to the provision of the present invention. As reported in JP 3-7231 A, 1α,3β-dihydroxy-20S-(3-hydroxy-3-methylbutyloxy)-9,10-seco-5,7,10(19)-pregnatriene is a vitamin D derivative effective as a therapeutic agent for hyperparathyroidism.

Namely, the present invention provides a therapeutic agent for cardiovascular complications associated with calcification resulting from the treatment of secondary hyperparathyroidism in a hemodialysis patient, which agent comprises 1α,3β-dihydroxy-20S-(3-hydroxy-3-methylbutyloxy)-9,10-seco-5,7,10(19)-pregnatriene as an active ingredient.

The cardiovascular complications are preferably selected from arteriosclerosis and ischemic heart disease.

The present invention also provides a prophylactic agent for cardiovascular complications associated with calcification resulting from the treatment of secondary hyperparathyroidism in a hemodialysis patient, which agent comprises 1α,3β-dihydroxy-20S-(3-hydroxy-3-methylbutyloxy)-9,10-seco-5,7,10(19)-pregnatriene as an active ingredient.

The cardiovascular complications are preferably selected from arteriosclerosis and ischemic heart disease.

According to another aspect of the present invention, there is provided the use of 1α,3β-dihydroxy-20S-(3-hydroxy-3-methylbutyloxy)-9,10-seco-5,7,10(19)-pregnatriene for the manufacture of a therapeutic agent for cardiovascular complications (e.g., arteriosclerosis, ischemic heart disease) associated with calcification resulting from the treatment of secondary hyperparathyroidism in a hemodialysis patient.

There is also provided the use of 1α,3β-dihydroxy-20S-(3-hydroxy-3-methylbutyloxy)-9,10-seco-5,7,10(19)-pregnatriene for the manufacture of a prophylactic agent for cardiovascular complications (e.g., arteriosclerosis, ischemic heart disease) associated with calcification resulting from the treatment of secondary hyperparathyroidism in a hemodialysis patient.

According to yet another aspect of the present invention, there is provided a treatment method of cardiovascular complications (e.g., arteriosclerosis, ischemic heart disease) associated with calcification resulting from the treatment of secondary hyperparathyroidism in a hemodialysis patient, which comprises administering to a patient in need of such treatment a therapeutically effective amount of 1α,3β-dihydroxy-20S-(3-hydroxy-3-methylbutyloxy)-9,10-seco-5,7,10(19)-pregnatriene.

There is also provided a prophylaxis method of cardiovascular complications (e.g., arteriosclerosis, ischemic heart disease) associated with calcification resulting from the treatment of secondary hyperparathyroidism in a hemodialysis patient, which comprises administering to a patient in need of such prophylaxis a therapeutically effective amount of 1α,3β-dihydroxy-20S-(3-hydroxy-3-methylbutyloxy)-9,10-seco-5,7,10(19)-pregnatriene.

This application claims the priority of Japanese Patent Application No. 2001-201673, the disclosure of which is hereby incorporated by reference in its entirety.

### BRIEF DESCRIPTION OF DRAWINGS

Figure 1 is a graph showing the Ca × Pi product in rat plasma. Each bar in the graph represents the mean ± SE. *** p<0.001 versus SNX control by Dunnett's multiple comparison test.

Figure 2 is a graph showing the Ca content in rat aorta. Each bar in the graph represents the mean ± SE. *** p<0.001 versus SNX control by Dunnett's multiple comparison test.

Figure 3 is a graph showing the Pi content in rat aorta. Each bar in the graph represents the mean ± SE. *** p<0.001 versus SNX control by Dunnett's multiple comparison test.

Figure 4 is a graph showing the Ca content in rat heart. Each bar in the graph represents the mean ± SE. *** p<0.001 versus SNX control by Dunnett's multiple comparison test. # p<0.05 by unpaired t-test.

Figure 5 is a graph showing the Pi content in rat heart. Each bar in the graph represents the mean ± SE. *** p<0.001 versus SNX control by Dunnett's multiple comparison test. ## p<0.01 by unpaired t-test.

Figure 6 is a graph showing the serum parathyroid hormone (PTH) levels. Each bar in the graph represents the mean ± SE. *** p<0.001 versus SNX control by Dunnett's multiple comparison test. # p<0.05 by unpaired t-test.

### PREFERRED MODE FOR CARRYING OUT THE INVENTION

As used herein, the term "vitamin D derivative" refers to a compound having the 9,10-secocholesta-5,7,10(19)-triene structure. It preferably refers to a compound having the (5Z,7E)-9,10-secocholesta-5,7,10(19)-triene structure. More preferably, it refers to a compound having the (1α,5Z,7E)-9,10-secocholesta-5,7,10(19)-trien-1-ol structure, still more preferably a compound having the (1α,5Z,7E)-9,10-secocholesta-5,7,10(19)-trien-1,25-diol structure, even more preferably a compound having the (1α,3β,5Z,7E)-9,10-secocholesta-5,7,10 (19)-trien-1,3-diol structure, and most preferably a compound having the (1α,3β,5Z,7E)-9,10-secocholesta-5,7,10(19)-trien-1,3,25-triol structure. All compounds having these structures can be identified as vitamin D derivatives. Such compounds may have any substituent at any position as long as they are capable of binding to vitamin D receptors.

1α,3β-Dihydroxy-20S-(3-hydroxy-3-methylbutyloxy)-9,10-seco-5,7,10(19)-pregnatriene as used herein is a known compound, which can be synthesized as described in, e.g., JP 61-267550 A.

As used herein, the term "treatment" or "treating" is intended to mean not only that symptoms are completely eliminated, but also that symptoms already developed are somewhat ameliorated, or that the progression or exacerbation of symptoms is delayed or reduced when compared to untreated patients.

The normal level of total serum calcium in humans will vary depending on techniques used for measurement, but usually ranges from 8.8 to 10.4 mg/dL (2.20 to 2.60 mmol/L). About 40% of the total blood calcium is bound to plasma proteins (primarily albumin). The remaining 60% is complexed with phosphate and citrate or present in the form of calcium ion. Meanwhile, most phosphorus in the body is complexed with oxygen as phosphate (PO₄). The body contains approximately 500 to 700 g of PO₄, 85% of which is contained in bone, where it is an important constituent of the crystalline hydroxyapatite. The normal plasma inorganic PO₄ concentration in adults will vary depending on techniques used for measurement, but usually ranges from 2.5 to 4.5 mg/dL (0.81 to 1.45 mmol/L).

There is a close relationship between calcium and phosphate metabolisms. The regulation of both Ca concentration equilibration and PO₄ concentration equilibration is influenced by the circulating blood levels of parathyroid hormone (PTH), vitamin D and calcitonin, etc. Although the Ca × Pi product in healthy adults is usually 30 (mg/dL)², a value exceeding 70 (mg/dL)² increases the risk of depositing calcium phosphate crystals in soft tissues (Am. J. Kidney Diseases, 31(4), 607-617, 1998).

As used herein, the phrase "not having an inducing action on cardiovascular complications associated with calcification" is intended to mean not only that a vitamin D derivative is completely free from the inducing action on cardiovascular complications associated with calcification, but also that such an inducing action is reduced when compared to the case of administering a pharmaceutical composition comprising other vitamin D derivative (e.g., 1,25-dihydroxyvitamin D₃). In other words, it is intended to mean that a vitamin D derivative does not have a risk of developing cardiovascular complications associated with calcification, or that a vitamin D derivative has a reduced risk when compared to 1,25-dihydroxyvitamin D₃. Preferably, it is intended to mean that a vitamin D derivative does not have an elevating action on serum Ca x Pi product beyond the normal range or does not cause elevation in serum Ca × Pi product before and after administration. Alternatively, it is also intended to mean that a vitamin D derivative results in reduced Ca and Pi deposition in the aorta and heart due to its short blood half-life, which is preferably shorter than that of 1,25-dihydroxyvitamin D₃ (e.g., less than 30 minutes).

In defining the phrase "having a prophylactic or improving action on cardiovascular complications associated with calcification" as used herein, the term "prophylactic" is intended to mean not only that symptoms are completely prevented from developing, but also that the severity or development of symptoms is reduced or delayed when compared to the case of not administering the pharmaceutical composition of the present invention. The term "improving" is intended to mean that symptoms already developed are somewhat ameliorated, or that the progression or exacerbation of symptoms is delayed or reduced when compared to the case of not administering the pharmaceutical composition of the present invention. In addition to these meanings, the term means complete elimination of symptoms. Thus, the phrase "having a prophylactic or improving action on cardiovascular complications associated with calcification" is preferably intended to mean that a vitamin D derivative does not have an elevating action on serum Ca × Pi product beyond the normal range or does not cause elevation in serum Ca × Pi product before and after administration. Alternatively, it is also intended to mean that a vitamin D derivative results in reduced Ca and Pi deposition in the aorta and heart due to its short blood half-life, which is preferably shorter than that of 1,25-dihydroxyvitamin D₃ (e.g., less than 30 minutes).

As used herein, the phrase "does not have an elevating action on serum Ca × Pi product beyond the normal range or does not cause elevation in serum Ca × Pi product before and after administration" is intended to mean in general that the serum Ca × Pi product is maintained at or below 70 (mg/dL)², preferably at or below 65 (mg/dL)², and more preferably at or below 60 (mg/dL)². It is preferable to have no elevating action on Ca × Pi product at a dose producing a therapeutic effect on secondary hyperparathyroidism, but it is also included to have a weak elevating action on Ca × Pi product.

As used herein, the phrase "having a weak elevating action on Ca × Pi product" is intended to mean that a vitamin D derivative has a weaker elevating action on serum Ca × Pi product than 1,25-dihydroxyvitamin D₃ at a dose producing the same level of therapeutic effect on secondary hyperparathyroidism as observed in 1,25-dihydroxyvitamin D₃.

Whether a vitamin D derivative has an elevating action on Ca × Pi product can be determined as described herein using an animal model, etc. For example, the serum Ca × Pi product is compared between drug-administered group receiving a specific type of vitamin D derivative and control group (non-administered). If the Ca × Pi product is significantly elevated in the drug-administered group when compared to the control group, the vitamin D derivative can be identified to have an elevating action on Ca × Pi product. Alternatively, the same comparative study may also be performed on patients in need of drug treatment for secondary hyperparathyroidism.

As used herein, the phrase "vitamin D derivative having a weak elevating action on Ca × Pi product" is intended to mean, for example, that there is a difference between the minimum dose required for a significant therapeutic effect on secondary hyperparathyroidism and the minimum dose required for a significant elevating action on Ca × Pi product. As used herein, the phrase "significant therapeutic effect on secondary hyperparathyroidism" is intended to mean a decrease in serum PTH levels.

More specifically, the dose of a vitamin D derivative is gradually increased to determine the following two doses: a dose at which the vitamin D derivative begins to produce a therapeutic effect on secondary hyperparathyroidism and a dose at which the vitamin D derivative begins to produce an elevating action on Ca × Pi product. It is desired that the difference between these two doses is preferably at least 5-fold, more preferably at least 10-fold, even more preferably at least 50-fold, and most preferably at least 100-fold. Alternatively, based upon a comparative study with 1,25-dihydroxyvitamin D₃, a vitamin D derivative can be identified to have a weak elevating action on Ca × Pi product if it shows a weaker elevating action on serum Ca × Pi product than 1,25-dihydroxyvitamin D₃ at a dose producing the same level of therapeutic effect on secondary hyperparathyroidism as observed in 1,25-dihydroxyvitamin D₃.

As used herein, the phrase "patient having the risk of developing cardiovascular complications" refers to a patient who is suspected to develop cardiovascular complications at high frequency in the future, more specifically a secondary hyperparathyroidism patient who is suspected to develop cardiovascular complications at high frequency in the future. Whether cardiovascular complications are developed at high frequency can be determined by statistical or epidemiological research. When viewed from a different angle, the phrase "patient having the risk of developing cardiovascular complications" as used herein includes patients having a serum Ca × Pi product of at least 60 (mg/dL)² or at least 65 (mg/dL)², particularly at least 70 (mg/dL)².

As long as cardiovascular complications are easily induced upon administration of 1,25-dihydroxyvitamin D₃, even patients having a serum Ca × Pi product less than 65 (mg/dL)² or less than 60 (mg/dL)² also fall within the scope of the "patient having the risk of developing cardiovascular complications" as used herein. Examples include chronic renal failure patients and hemodialysis patients, as well as patients undergoing treatment with 1,25-dihydroxyvitamin D₃, particularly, in order to treat secondary hyperparathyroidism.

In defining the term "therapeutic agent" as used herein, the term "therapeutic" is intended to mean not only that symptoms are completely eliminated, but also that symptoms already developed are somewhat ameliorated, or that the progression or exacerbation of symptoms is delayed or reduced when compared to the case of not administering the therapeutic agent of the present invention.

In defining the term "prophylactic agent" as used herein, the term "prophylactic" is intended to mean not only that symptoms are completely prevented from developing, but also that the severity or development of symptoms is reduced or delayed when compared to the case of not administering the prophylactic agent of the present invention.

In the treatment method and the therapeutic or prophylactic agent of the present invention, administration may be accomplished in any manner, for example, via oral or parenteral (e.g., intravenous, intramuscular, intraperitoneal) routes.

To prepare the pharmaceutical composition of the present invention, a vitamin D derivative as an active ingredient is preferably formulated into a suitable dosage form, if desired, in combination with pharmaceutically acceptable carriers, excipients, disintegrating agents, lubricants, binders, flavoring agents, coloring agents and the like. Likewise, to prepare the therapeutic or prophylactic agent of the present invention, an active ingredient 1α,3β-dihydroxy-20S-(3-hydroxy-3-methylbutyloxy)-9,10-seco-5,7,10(19)-pregnatriene is preferably formulated into a suitable dosage form, if desired, in combination with pharmaceutically acceptable carriers, excipients, disintegrating agents, lubricants, binders, flavoring agents, coloring agents and the like. Any dosage form may be employed for this purpose, including tablets, granules, fine granules, capsules, powders, injections, solutions, suspensions, emulsions, percutaneous absorption formulations and suppositories.

The dose of the pharmaceutical composition or therapeutic or prophylactic agent of the present invention can be selected as appropriate for the type of disease to be treated, the condition, physique, diathesis, age and sex of a patient, the intended route of administration, or the type of dosage form, etc. The dose as the active ingredient ranges usually from 0.001 to 1000 µg/kg/day, preferably from 0.01 to 100 µg/kg/day, and more preferably from 0.1 to 10 µg/kg/day.

### EXAMPLES

The present invention will be further described in the following Examples, which are not intended to limit the scope of the invention.

Rats with 5/6 nephrectomy (5/6 nephrectomized rats) were used as an experimental model to examine the agent of the present invention for its effect on cardiovascular complications. Such 5/6 nephrectomized rats are widely used as an experimental nephritis model with 2HPT (Ichikawa et al., Nephrology, vol. 4, pp. 391-395, 1998).

### 1. Experimental method

Seven-week-old male SD rats (Japan SLC, Inc.) were subjected to 5/6 nephrectomy according to the method of Platt et al. (Platt R. et al., Clin. Sci. 11, 217-231, 1952) to remove the whole of the right kidney and 2/3 of the left kidney. The rats were then allowed to rest for 10 weeks to create a kidney failure model with 2HPT.

The nephrectomized rats were divided into 4 test groups: OCT 1.25 µg/kg group (8 rats), OCT 6.25 µg/kg group (8 rats), 1,25(OH)₂D₃ 0.125 µg/kg group (8 rats) and 1,25(OH)₂D₃ 0.625 µg/kg group (8 rats).

Starting at 10 weeks after surgery, the rats of each group were administered with a given dose of OCT or 1,25-dihydroxyvitamin D₃ (each dissolved in phosphate buffer, pH 6.52) via the tail vein. Administration was conducted three times a week for a total of 7 administration (i.e., on Monday, Wednesday, Friday, Monday, Wednesday, Friday and Monday). Twenty-four hours after the last administration, the blood was collected from the abdominal descending aorta of each rat under ether anesthesia. The rats were then sacrificed by exsanguination and the heart and aorta were excised from each rat.

Six rats of sham-operated (Sham control) group were subjected to renal decapsulation alone instead of 5/6 nephrectomy and then treated in the same manner as used for the test groups, except that phosphate buffer (Ph 6.52) was administered instead of OCT or 1,25-dihydroxyvitamin D₃. Likewise, 9 rats of SNX control group were treated in the same manner as used for the test groups, except that phosphate buffer (pH 6.52) was administered instead of OCT or 1,25-dihydroxyvitamin D₃.

### 2. Ca and Pi in serum

After serum was separated from the collected blood; Ca and Pi contained in the serum were measured with a Hitachi 7170-E autoanalyzer using an Autosera reagent (Daiichi Pure Chemicals Co., Ltd.) to determine the serum Ca × Pi product for each rat.

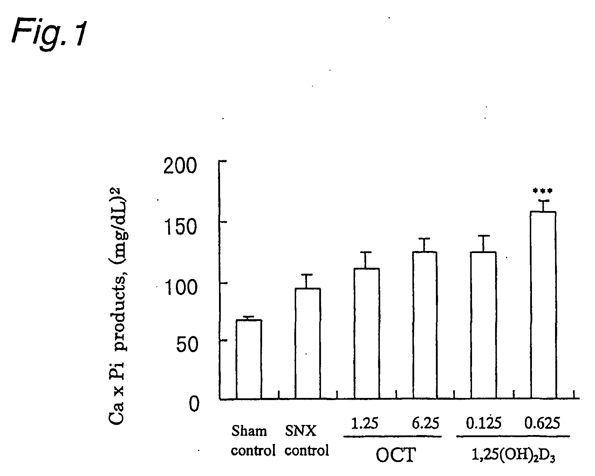

The results obtained are shown in Figure 1. The relationship between Sham control group and SNX control group was analyzed by unpaired t-test. The relationship between SNX control group and each OCT- or 1,25(OH)₂D₃- administered group was analyzed by Dunnett's multiple comparison test.

As shown in Figure 1, the 1,25(OH)₂D₃ 0.625 µg/kg group showed a significant elevation in serum Ca × Pi product over the SNX control group (p<0.001). The OCT 6.25 µg/kg group and the 1,25(OH)₂D₃ 0.125 µg/kg group showed almost the same level.

### 3. Ca and Pi contents in individual organs

Each heart and aorta were lyophilized for 24 hours in a FREEZE DRYER FD-5N (trade name, EYELA) and then treated for 48 hours with a chloroform/methanol solution (chloroform:methanol = 2:1, special-reagent-grade chloroform and methanol were both purchased from Junsei Chemical Co., Ltd.). Subsequently, each organ was dehydrated for 3 hours with acetone (special reagent grade, Junsei Chemical Co., Ltd.) and then allowed to stand at room temperature for 24 hours. After delipidization and dehydration, each organ was transferred to a crucible and weighed to determine dry weight (Mettler). Ashing was carried out in a muffle furnace KDF S90 (trade name, Denken Co., Ltd., Kyoto, Japan) at 900°C for 12 hours. The ashed sample was mixed with 36 mol/L hydrochloric acid (1.0 mL), allowed to stand for 3 hours to dissolve sufficiently, and then collected in a polypropylene tube. The crucible was washed with purified water (1.0 mL), which was then also collected in the tube. Each sample was stored at 4°C until measurement. Ca and Pi were measured with a Hitachi 7170-E autoanalyzer to calculate the Ca and Pi contents per gram of each organ.

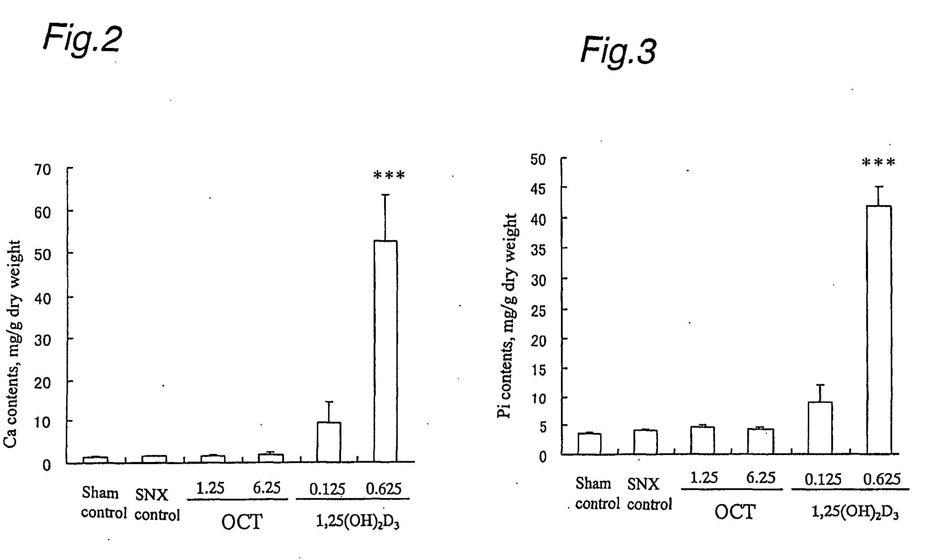

The results obtained are shown in Figures 2 to 5. The relationship between Sham control group and SNX control group was analyzed by unpaired t-test. The relationship between SNX control group and each OCT- or 1,25(OH)₂D₃-administered group was analyzed by Dunnett's multiple comparison test.

As shown in Figures 2 and 3, the administration of 1,25-dihydroxyvitamin D₃ caused dose-dependent significant increases in Ca and Pi contents in the aorta over the SNX control group (p<0.001). When a comparison was made between OCT 6.25 µg/kg group and 1,25(OH)₂D₃ 0.125 µg/kg group, both of which showed almost the same level of serum Ca × Pi product, it was indicated that 1,25-dihydroxyvitamin D₃ resulted in apparently higher deposition of Ca and Pi in the aorta as compared to OCT.

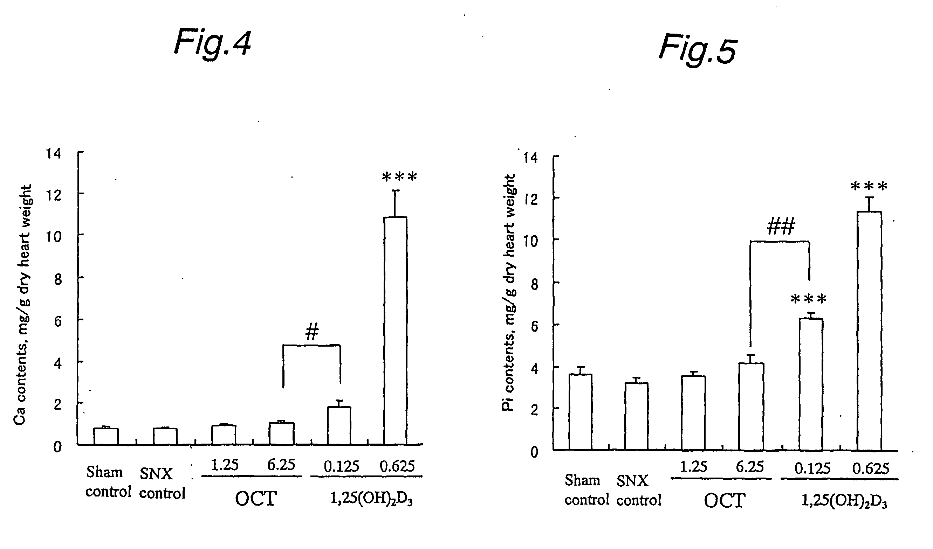

As shown in Figures 4 and 5, the administration of 1,25-dihydroxyvitamin D₃ caused dose-dependent significant increases in Ca and Pi contents in the heart over the SNX control group (p<0.001). When a comparison was made between OCT 6.25 µg/kg group and 1,25(OH)₂D₃ 0.125 µg/kg group, both of which showed almost the same level of serum Ca × Pi product, it was indicated that 1,25-dihydroxyvitamin D₃ resulted in significantly higher deposition of Ca and Pi in the heart as compared to OCT (Ca: p<0.05; Pi: p<0.01).

### 4. Parathyroid hormone levels in blood

After serum was separated from the collected blood, the level of parathyroid hormone (PTH) contained in the serum was measured with a commercially available radioimmunoassay kit (Immutopics, Inc., USA).

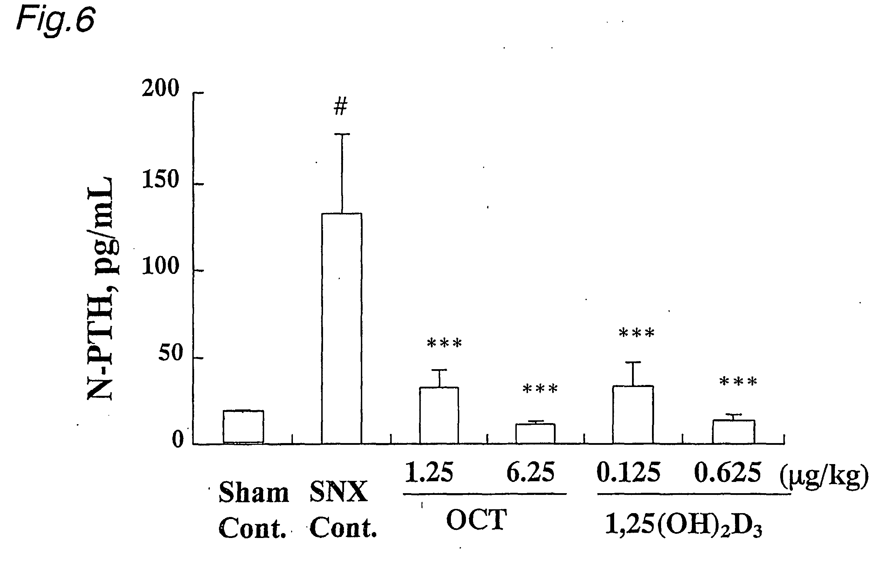

The results obtained are shown in Figure 6. The relationship between Sham control group and SNX control group was analyzed by unpaired t-test. The relationship between SNX control group and each OCT- or 1,25(OH)₂D₃- administered group was analyzed by Dunnett's multiple comparison test.

As shown in the figure, 1,25-dihydroxyvitamin D₃ and OCT both had a decreasing effect on PTH levels at all doses, showing their usefulness as therapeutic agents for secondary hyperparathyroidism. When compared to Figure 1, this result indicates that 1,25-dihydroxyvitamin D₃ causes no significant elevation in Ca × Pi product at a dose of 0.125 µg/kg, whereas it causes a significant elevation at a dose of 0.625 µg/kg. On the other hand, the therapeutic effect on secondary hyperparathyroidism, i.e., the decreasing effect on PTH levels was observed at a dose of either 0.125 or 0.625 µg/kg. Thus, the minimum dose of 1,25-dihydroxyvitamin D₃ required to produce an elevating action on Ca × Pi product is 0.625 µg/kg, and the therapeutic effect on secondary hyperparathyroidism can be produced at a dose of 0.125 µg/kg. Taken together, these observations indicate that there is only a minimum of 5-fold difference between these two doses. In the case of OCT, no significant elevating action on Ca × Pi product could be observed at a dose of either 1.25 or 6.25 µg/kg, whereas the decreasing effect on PTH levels was observed at a dose of either 1.25 µg/kg or 6.25 µg/kg. Thus, there is at least a 5-fold (estimatively at least 25-fold) difference between the doses of OCT required to produce a decreasing effect on PTH levels and an elevating action on Ca × Pi product.

Elevated blood Ca × Pi product is one of the major factors for calcification of soft tissues, but other various factors are also involved. For example, vitamin D derivatives generally have a stimulatory effect on intracellular uptake of calcium and phosphate and, in turn, typically promote calcification. However, vitamin D derivatives free from these effects may not promote cardiovascular calcification. Alternatively, in view of the fact that calcification occurs in blood vessels, vitamin D derivatives having a short blood retention time may also not promote cardiovascular calcification.

The OCT used in the above examples has a blood half-life of about 20 minutes and rapidly disappears from the blood when compared to 1,25-dihydroxyvitamin D₃ whose blood half-life is 1 hour or longer. Such a difference in half-life may also contribute to the result that the OCT-administered groups showed significantly lower deposition of Ca and Pi in the aorta and heart than the 1,25(OH)₂D₃- administered groups. Thus, reduced Ca and Pi deposition in the aorta and heart can be expected for vitamin D derivatives having a short blood half-life, which is preferably shorter than that of 1,25-dihydroxyvitamin D₃ (e.g., less than 30 minutes).

### INDUSTRIAL APPLICABILITY

As stated above, the pharmaceutical composition of the present invention is useful in treating secondary hyperparathyroidism because it can reduce the risk of inducing cardiovascular complications associated with calcification. Likewise, since it is evident that the risk of calcification is lower in the therapeutic agent of the present invention than in 1,25-dihydroxyvitamin D₃, the therapeutic agent of the present invention is useful as a therapeutic or prophylactic agent for cardiovascular complications resulting from the treatment of secondary hyperparathyroidism in a hemodialysis patient.

## Claims

1. A pharmaceutical composition for the treatment of secondary hyperparathyroidism, comprising a vitamin D derivative as an active ingredient, said vitamin D derivative not having an inducing action on cardiovascular complications associated with calcification.

2. A pharmaceutical composition for the treatment of secondary hyperparathyroidism, comprising a vitamin D derivative as an active ingredient, said vitamin D derivative having a prophylactic or improving action on cardiovascular complications associated with calcification resulting from the treatment of secondary hyperparathyroidism.

3. The pharmaceutical composition of claim 1 or 2, wherein the vitamin D derivative has a weak elevating action on Ca × Pi product.

4. The pharmaceutical composition of claim 3, wherein the vitamin D derivative is 1α,3β-dihydroxy-20S-(3-hydroxy-3-methylbutyloxy)-9,10-seco-5,7,10(19)-pregnatriene.

5. A treatment method of secondary hyperparathyroidism, comprising administering the pharmaceutical composition of any one of claims 1 to 4 to a patient in need of such treatment.

6. The method of claim 5, wherein the patient has the risk of developing cardiovascular complications associated with calcification.

7. A pharmaceutical composition for the treatment of secondary hyperparathyroidism, comprising a vitamin D derivative as an active ingredient, and being administered to a patient having the risk of developing cardiovascular complications associated with calcification.

8. A therapeutic agent for cardiovascular complications associated with calcification resulting from the treatment of secondary hyperparathyroidism in a hemodialysis patient, comprising 1α,3β-dihydroxy-20S-(3-hydroxy-3-methylbutyloxy)-9,10-seco-5,7,10(19)-pregnatriene as an active ingredient.

9. The therapeutic agent of claim 8, wherein the cardiovascular complication is arteriosclerosis or ischemic heart disease.

10. A prophylactic agent for cardiovascular complications associated with calcification resulting from the treatment of secondary hyperparathyroidism in a hemodialysis patient, comprising 1α,3β-dihydroxy-20S-(3-hydroxy-3-methylbutyloxy)-9,10-seco-5,7,10(19)-pregnatriene as an active ingredient.

11. The prophylactic agent of claim 10, wherein the cardiovascular complication is arteriosclerosis or ischemic heart disease.

12. The use of 1α,3β-dihydroxy-20S-(3-hydroxy-3-methylbutyloxy)-9,10-seco-5,7,10(19)-pregnatriene for the manufacture of a prophylactic or therapeutic agent for cardiovascular complications associated with calcification resulting from the treatment of secondary hyperparathyroidism in a hemodialysis patient.

13. A prophylactic or treatment method of cardiovascular complications associated with calcification resulting from the treatment of secondary hyperparathyroidism in a hemodialysis patient, comprising administering to a patient in need of such treatment a therapeutically effective amount of 1α,3β-dihydroxy-20S-(3-hydroxy-3-methylbutyloxy)-9,10-seco-5,7,10(19)-pregnatriene.
